# EUROPEAN PATENT APPLICATION

(11) **EP 4 023 160 A1**
(43) Date of publication of application: **06.07.2022**
(21) Application number: 19943467.1
(22) Date of filing: 27.08.2019
(51) Int. Cl.: A61B 10/00, A61N 5/06

(54) **IMAGING DEVICE, TREATMENT DEVICE, AND IMAGING METHOD**

(71) Applicant: Shimadzu Corporation, Kyoto-shi Kyoto 604-8511 (JP)
(72) Inventor: TSUMATORI Hiroyuki, Kyoto-shi, Kyoto 604-8511 (JP)
(74) Representative: Müller-Boré & Partner Patentanwälte PartG mbB
(86) International application number: PCT/JP2019/033591
(87) International publication number: WO 2021/038734

(57) **Abstract**

An imaging apparatus (1) includes: an excitation light source (242) that irradiates a treating area of a subject (ST), to which a treatment drug including the fluorescent pigment has been administered, with excitation light that excites a fluorescent pigment; a first imaging unit (28) that images the treating area and acquires a visible moving image; a second imaging unit (29) that acquires a fluorescent image by imaging fluorescent light generated from the fluorescent pigment of the treating area when irradiating with the excitation light; a composite image generating unit (19) that generates a composite image by superimposing the fluorescent image on the visible moving image; and an image display unit (15) that displays the composite image. The excitation light source irradiates with the excitation light having a predetermined pulse width.

## Description

### TECHNICAL FIELD

The present invention relates to an imaging apparatus, a therapeutic apparatus, and an imaging method.

### BACKGROUND ART

In recent years, photoimmunotherapy is attracting attention as a cancer treatment method. With photoimmunotherapy, a treatment drug (RM-1929) that is a combination of a fluorescent reagent (IR700) and an epidermal growth factor receptor (EGFR) antibody is injected into a subject. When the treatment drug is injected into the subject, the antibody bonds with cancer cell surfaces. It is said that if the subject is irradiated with near infrared light of approximately 600 to 700 nm in this state for a predetermined period of time, heat generates in the treatment drug (RM-1929), cancer cells are destroyed, and a ligand portion or hydrophilic group of IR700 is detached, thereby forming an aggregate and quenching the light (Non-Patent Documents 1 and 2).

According to such photoimmunotherapy, acquisition of a fluorescent image during treatment is important since treatment position and therapeutic results (that is, progression of treatment) can be confirmed by detecting fluorescent light derived from the fluorescent reagent (IR700) to be used.

### Prior Art Documents

### Non-Patent Documents

Non-Patent Document 1 : K. Sato et al., 'Photo-induced ligand release from a silicon phthalocyanine dye conjugated with monoclonal antibodies; A mechanism of cancer cell cytotoxicity after near infrared photoimmunotherapy', ACS Central Science, Nov. 7, 2018
Non-Patent Document 2 : Shimadzu Corporation, 'First time to successfully develop a light remote control switch for killing only targeted cells -Expectations for contribution to cancer treatment with little side effects-', [online], [Searched March 10, 2019] Internet <URL:https//WWW.SHIMADZU.CO.JP/news/press/9qbnqudlmddlrfby.html>

### DISCLOSURE OF THE INVENTION

### Problems to be Solved by the Invention

However, with such photoimmunotherapy, there is a problem that if irradiating with near infrared light so as to confirm treatment position and therapeutic results, reaction to the treatment drug at the irradiated position progresses, and treatment progresses unintentionally. When treatment progresses unintentionally, a problem that sufficient therapeutic results cannot be achieved occurs.

The present invention is devised to solve these problems, and aims to provide an imaging apparatus, a therapeutic apparatus, and an imaging method capable of accurately acquiring treatment position and therapeutic results without progression of the treatment drug reaction (that is, without progression of treatment).

### Means of Solving the Problems

A first aspect of the present invention relates to an imaging apparatus, including: an excitation light source that irradiates a treating area of a subject, to which a treatment drug including the fluorescent pigment has been administered, with excitation light that excites a fluorescent pigment; a first imaging unit that images the treating area and acquires a visible moving image; a second imaging unit that acquires a fluorescent image by imaging fluorescent light generated from the fluorescent pigment of the treating area when irradiating with the excitation light; a composite image generating unit that generates a composite image by superimposing the fluorescent image on the visible moving image; and an image display unit that displays the composite image. The excitation light source irradiates with the excitation light having a predetermined pulse width.

A second aspect of the present invention relates to an imaging method, including the steps of: irradiating a treating area of a subject, to which a treatment drug including the fluorescent pigment has been administered, with an excitation light that excites a fluorescent pigment; imaging the treating area and acquiring a visible moving image; acquiring a fluorescent image by imaging fluorescent light generated from the fluorescent pigment of the treating area when irradiating with the excitation light; generating a composite image by superimposing the fluorescent image on the visible moving image; and displaying the composite image. The step of irradiating with an excitation light irradiates with the excitation light having a predetermined pulse width.

### Results of the Invention

According to the present invention, an imaging apparatus, a therapeutic apparatus, and an imaging method capable of accurately acquiring treatment position and therapeutic results without progression of the treatment drug reaction (that is, without progression of treatment) are realized.

### BRIEF DESCRIPTIONS OF DRAWINGS

FIG. 1 is a perspective view illustrating an embodiment of an imaging apparatus of the present invention;
FIG. 2 is a side view of the imaging apparatus of FIG. 1;
FIG. 3 is a plan view of the imaging apparatus of FIG. 1;
FIG. 4 is a block diagram illustrating a main control system of the imaging apparatus of FIG. 1;
FIG. 5 is a flowchart of an imaging program executed by the imaging apparatus of FIG. 1;
FIG. 6 shows exemplary images to be displayed on an image display unit included in the imaging apparatus of FIG. 1;
FIG. 7 is a timing chart illustrating a relationship between a second light source of the imaging apparatus of FIG. 1, the operation of an excitation light switch (operation unit), and the frame rate of an excitation light sensor;
FIG. 8 is a timing chart illustrating a relationship between the second light source of an imaging apparatus according to Modified Example 1 of the present invention, the operation of the excitation light switch (operation unit), and the frame rate of the excitation light sensor; and
FIG. 9 is a block diagram illustrating a main control system of an imaging apparatus according to Modified Example 2 of the present invention.

### DESCRIPTION OF EMBODIMENT

An imaging apparatus and an imaging method according to the present invention are described in detail below based on a preferred embodiment illustrated in the attached drawings.

### <Embodiment>

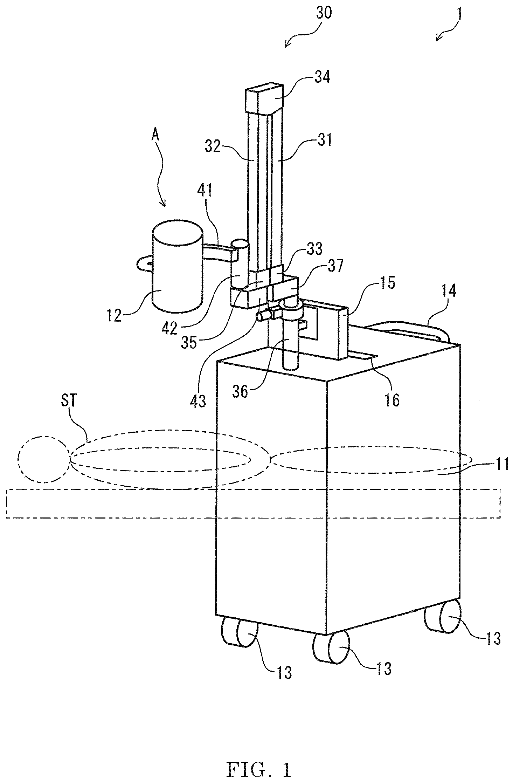

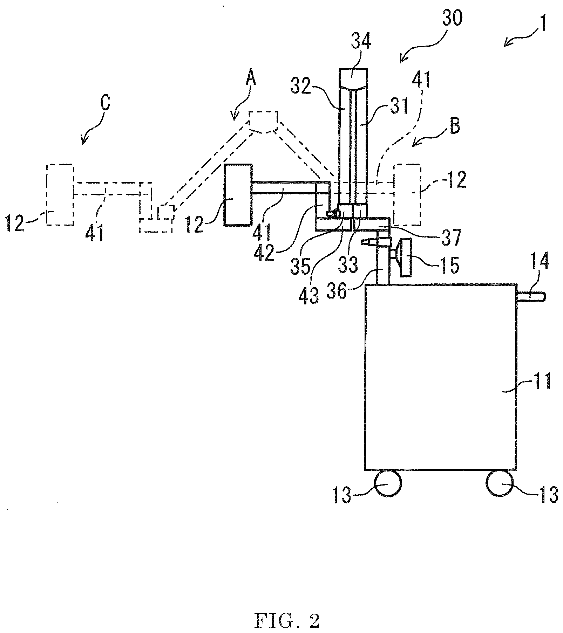

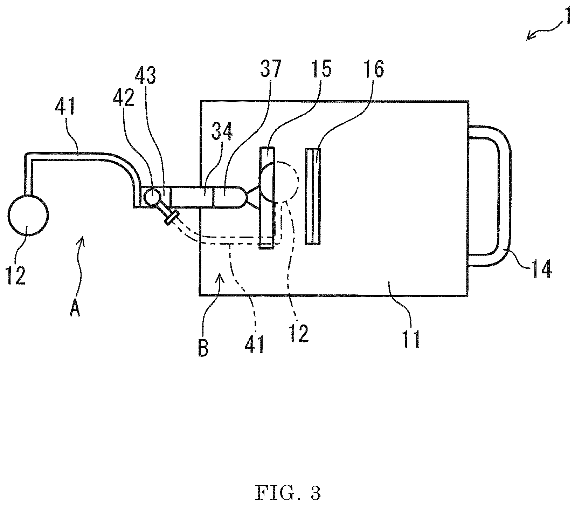

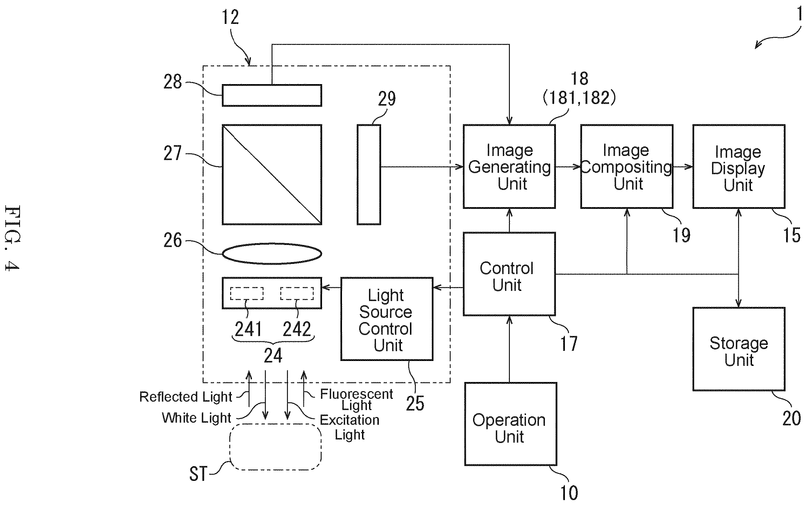

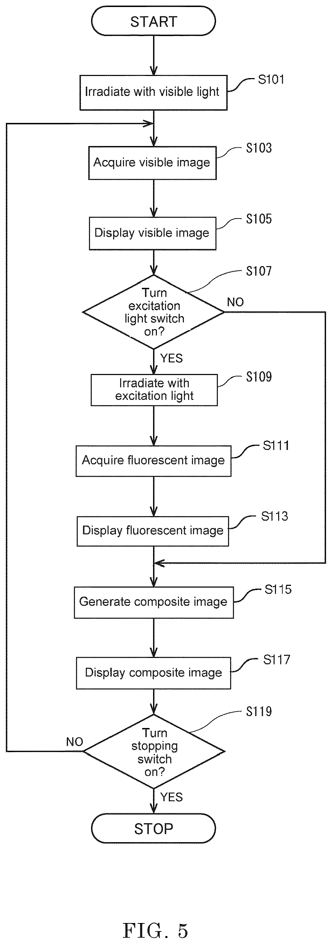

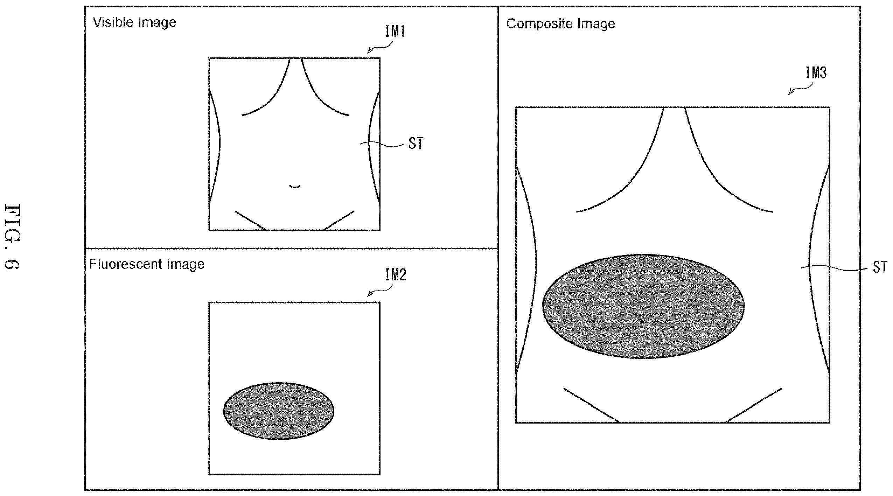

FIG. 1 is a perspective view illustrating an embodiment of an imaging apparatus of the present invention. FIG. 2 is a side view of the imaging apparatus of FIG. 1. FIG. 3 is a plan view of the imaging apparatus of FIG. 1. FIG. 4 is a block diagram illustrating a main control system of the imaging apparatus of FIG. 1. FIG. 5 is a flowchart of an imaging program executed by the imaging apparatus of FIG. 1. FIG. 6 shows exemplary images to be displayed on an image display unit included in the imaging apparatus of FIG. 1. Note that for convenience of explanation, the upper sides of FIGS. 1, 2 and 6 are called 'top (or top part)' and the lower sides thereof are called 'bottom (or bottom part)' hereafter.

An imaging apparatus 1 illustrated in FIG. 1 is an apparatus for irradiating with excitation light a treatment drug (RM-1929) that is a combination of an epidermal growth factor receptor (EGFR) antibody and a fluorescent reagent (IR700) injected into a subject ST, and imaging the fluorescent light emitted from the fluorescent reagent (IR700). Use of the imaging apparatus 1 allows confirmation of treatment position and therapeutic results of the subject ST, for example (that is, progression of treatment), when carrying out photoimmunotherapy for the subject ST as described later.

The imaging apparatus 1 includes a cart 11 provided with four wheels 13, an arm mechanism 30 deployed near the front of the top surface of the cart 11 in the running direction (leftward direction in FIG. 2 and FIG. 3), a lighting and imaging unit 12 deployed on the arm mechanism 30 via a sub-arm 41, and an image display unit 15 that is a monitor. A handle 14 to be used when moving the cart 11 is attached to the back of the cart 11 in the running direction thereof. Furthermore, a recess 16 for mounting a remote control, which is for remotely operating the imaging apparatus 1, is formed in the top surface of the cart 11.

Moreover, while details will be described later, when power of the imaging apparatus 1 is turned on, an imaging program is executed (that is, each step given in FIG. 5 is carried out), and a visible image IM1, a fluorescent image IM2 and a composite image IM3 are displayed on the image display unit 15 (FIG. 6).

The arm mechanism 30 described above is deployed on the front side of the cart 11 in the running direction thereof. The arm mechanism 30 includes a first arm member 31 connected via a hinge 33 to a support part 37, which is installed on a column 36 standing on the front side of the cart 11 in the running direction thereof. The first arm member 31 is swingable with respect to the cart 11 via the column 36 and the support part 37 by virtue of the action of the hinge part 33. Note that the image display unit 15 described above is attached to the column 36.

A second arm member 32 is connected to an upper end of the first arm member 31 via a hinge part 34. The second arm member 32 is swingable with respect to the first arm member 31 by virtue of the action of the hinge part 34. This allows the first arm member 31 and the second arm member 32 take a photographing posture open at a predetermined angle centering around the hinge part 34, which is a part connecting the first arm member 31 and the second arm member 32, as indicated by virtual lines given a reference character C in FIG. 2, and a standby posture where the first arm member 31 and the second arm member 32 are near each other, as indicated by solid lines given a reference character A in FIGS. 1 to 3.

A support part 43 is connected to a lower end of the second arm member 32 via a hinge part 35. The support part 43 is swingable with respect to the second arm member 32 by virtue of the action of the hinge part 35. A rotating shaft 42 is supported by the support part 43. The sub-arm 41 supporting the lighting and imaging unit 12 rotates around the rotating shaft 42 that is installed at the front end of the second arm member 32. As a result, the lighting and imaging unit 12 moves, due to rotation of the sub-arm 41, between a position on the front side of the arm mechanism 30 in the running direction of the cart 11 for taking the photographing posture or the standby posture, as indicated by the virtual lines given the reference character C in FIG. 2 or the solid lines given the reference character A in FIGS. 1 to 3, and a position on the back side of the arm mechanism 30 having the posture when moving the cart 11, in the running direction of the cart 11, as indicated by virtual lines given a reference character B in FIG. 2 and FIG. 3.

As illustrated in FIG. 4, the lighting and imaging unit 12 includes a light source 24, a light source control unit 25, a zoom lens 26, a prism 27, a white light sensor 28, and an excitation light sensor 29. When using the imaging apparatus 1, it is preferable to have the lighting and imaging unit 12 at a distance of approximately several tens of centimeters from a diseased part of the subject ST.

The light source 24 includes a first light source 241, which is a white light source, and a second light source 242, which is an excitation light source. When the first light source 241 is turned on, the subject ST is irradiated with white light, and the white light sensor 28 detects reflected white light reflected from the subject ST.

The second light source 242 irradiates with excitation light for exciting the fluorescent reagent (IR700). When the second light source 242 is turned on, the subject ST is irradiated with near infrared light (excitation light) of a wavelength 600 to 700 nm, exciting the fluorescent reagent (IR700) of the treatment drug (RM-1929) administered to the subject ST. When the fluorescent reagent (IR700) is excited, near infrared light having a peak of approximately 700 or 770 nm emits as fluorescent light and is detected by the excitation light sensor 29. Note that since if irradiating with excitation light over a long period of time, reaction of the fluorescent reagent (IR700) progresses and treatment progresses unintentionally, pulse-lighting occurs for only a duration corresponding to the imaging time of one field (e.g., 16 msec) of the excitation light sensor 29 (details will be described later) according to this embodiment.

The light source control unit 25 has a function of controlling turning on of the first light source 241. This function allows the first light source 241 to irradiate with the white light and stop irradiation. Furthermore, the light source control unit 25 has a function of controlling turning on of the second light source 242. This function allows the second light source 242 to irradiate with the excitation light and stop irradiation. Note that the light source control unit 25 is connected to the control unit 17 for controlling the entire imaging apparatus 1, and controls turning on of the first light source 241 and the second light source 242 according to instructions from the control unit 17.

The reflected light (white light) reflected from the subject ST and the fluorescent light generated by the fluorescent reagent (IR700) within the subject ST are incident on the zoom lens 26. The reflected light (white light) is formed into an image by the white light sensor 28 and the fluorescent light is formed into an image by the excitation light sensor 29 using the zoom lens 26. Light from the zoom lens 26, namely white light and fluorescent light, is incident on the prism 27; however, the white light and the fluorescent light incident on the prism 27 are separated by the prism 27, the white light is directed toward the white light sensor 28, and the fluorescent light is directed toward the excitation light sensor 29.

The white light sensor 28 is an imaging element for detecting a part of the reflected light (white light) separated by the prism 27, forming a visible image of the subject ST into an image at an NTSC (National Television System Committee) frame rate (30 frames/sec (60 fields/sec)), for example. Furthermore, the excitation light sensor 29 is an imaging element for detecting a part of the near infrared light (fluorescent light) separated by the prism 27, forming a fluorescent image of the subject ST into an image at the NTSC frame rate (30 frames/sec (60 fields/sec)).

Moreover, as illustrated in FIG. 4, the imaging apparatus 1 includes the control unit 17, an image generating unit 18, an image compositing unit 19, a storage unit 20, and an operation unit 10. The respective units are arranged on the cart 11.

The control unit 17 is configured by a CPU for executing logical operation, a ROM in which an operation program required for control of the apparatus is stored, and a RAM in which data etc. is temporarily stored during control etc., and has a function of controlling the entire apparatus. The control unit 17 is electrically connected to the light source control unit 25, the image generating unit 18, the image compositing unit 19, the image display unit 15, the storage unit 20, and the operation unit 10, and reads out an imaging program stored in the storage unit 20 so as to control the respective units when the power of the imaging apparatus 1 is turned on.

The reflected light (white light) detected by the white light sensor 28 and the near infrared light (fluorescent light) detected by the excitation light sensor 29 are input to the image generating unit 18. The image generating unit 18 then generates a 24-bit (= 3×8) visible image IM1 configured by three colors RGB (red, green and blue) from the reflected light (white light) that is detected by the white light sensor 28. Furthermore, the image generating unit 18 generates an 8-bit fluorescent image IM2 from the near infrared light (fluorescent light) that is detected by the excitation light sensor 29. According to the embodiment, the image generating unit 18 functions as a first imaging unit 181, which acquires a visible image IM1 by imaging at the NTSC frame rate the subject ST irradiated with the white light, and a second imaging unit 182, which acquires a fluorescent image IM2 by imaging at the NTSC frame rate the fluorescent light generated by the fluorescent reagent (IR700).

The image compositing unit 19 composites the visible image IM1 and the fluorescent image IM2 generated by the image generating unit 18 so as to generate a composite image IM3. As illustrated in FIG. 6, according to the embodiment, the visible image IM1, the fluorescent image IM2, and the composite image IM3 are displayed collectively on the image display unit 15. Then, a doctor is capable of accurately acquiring treatment position and therapeutic results of the subject ST (that is, progression of treatment) through observation of the composite image IM3.

The storage unit 20 is structured so as to store the imaging program to be executed by the control unit 17, and the fluorescent image IM2 generated by the image generating unit 18, etc.

The operation unit 10 is a user interface for operating the imaging apparatus 1. For example, the operation unit 10 is structured so as to operate, such as allow the light source 24 to irradiate with light, stop irradiation, adjust brightness and sensitivity, and select a method of displaying an image on the image display unit 15.

Next, the imaging program to be executed by the control unit 17 is described while referencing FIG. 5. The imaging program is read out from the storage unit 20 once the power of the imaging apparatus 1 is turned on, and executed by the control unit 17.

As shown in FIG. 5, when the imaging program is executed, the control unit 17 controls the light source control unit 25 so as to turn on the first light source 241. When the first light source 241 is turned on, the subject ST is irradiated with white light. Once processing of step S101 is completed, processing progresses to step S103.

In step S103, the control unit 17 controls a first imaging unit 181 of the image generating unit 18 so as to acquire a visible image IM1 from data of the white light sensor 28 to be input to the image generating unit 18 at the NTSC frame rate. Once processing of step S103 is completed, processing progresses to step S105.

In step S105, the control unit 17 controls the image compositing unit 19 and the image display unit 15 so as to send the visible image IM1 acquired in step S103 to the image compositing unit 19, and display the visible image IM1 on the image display unit 15. Once processing of step S105 is completed, processing progresses to step S107.

In step S107, the control unit 17 determines whether or not an excitation light switch (switch for irradiating with excitation light) of the operation unit 10 has been turned on. When the excitation light switch has been turned on, processing progresses to step S109, and when the excitation light switch is off, processing progresses to step S115.

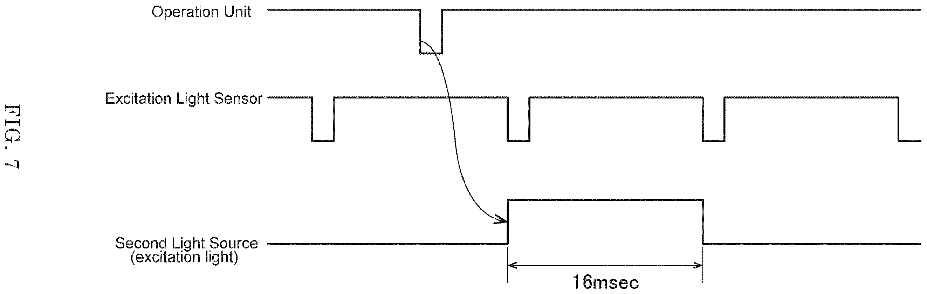

In step S109, the control unit 17 controls the light source control unit 25 so as to make the second light source 242 pulse-light. FIG. 7 is a timing chart illustrating a relationship between the timing of pulse-lighting of the second light source 242 according to the embodiment, timing of the operation of the excitation light switch (operation unit 10), and the frame rate of the excitation light sensor 29. Note that in FIG. 7, the timing of when the excitation light switch is turned on is indicated on the operation unit 10 with a negative logic pulse signal, end of each field of the excitation light sensor 29 is indicated with a negative logic pulse signal, and timing of turning on the second light source 242 is indicated with a positive logic pulse signal. As shown in FIG. 7, the excitation light sensor 29 outputs an image signal for each field at 60 Hz (16 msec cycle). Then, when it is determined in step S107 that the excitation light switch has been turned on, the control unit 17 makes the second light source 242 turn on for 16 msec (that is, duration of one field) synchronized with the next field (or frame) of the excitation light sensor 29. When the second light source 242 starts pulse-lighting in this manner, the subject ST is irradiated with excitation light. Once processing of step S109 is completed, processing progresses to step S111.

In step S111, the control unit 17 controls a second imaging unit 182 of the image generating unit 18 so as to acquire a fluorescent image IM2 from data generated by the excitation light sensor 29 to be input to the image generating unit 18 at the NTSC frame rate. More specifically, according to the embodiment, since the second light source 242 pulse-lights only for the duration of one field in step S109, the fluorescent image IM2 for this duration is acquired and stored in the storage unit 20. Once processing of step S111 is completed, processing progresses to step S113.

In step S113, the control unit 17 controls the image compositing unit 19 and the image display unit 15 so as to send the fluorescent image IM2 acquired and stored in step S111 to the image compositing unit 19, and display the fluorescent image IM2 on the image display unit 15. In this manner, the embodiment is structured so as to make the excitation light pulse-light for only a duration corresponding to the imaging time (e.g., 16 msec) of one frame of the excitation light sensor 29 and acquire the fluorescent image IM2, resulting in preventing irradiation with the excitation light over a long period of time, in steps S109 to S113. Once processing of step S113 is completed, processing progresses to step S115.

In step S115, the control unit 17 controls the image compositing unit 19 so as to superimpose and composite the visible image IM1 acquired in step S103 and the fluorescent image IM2 acquired in step S111, and generate a composite image IM3. Once processing of step S115 is completed, processing progresses to step S117.

In step S117, the control unit 17 controls the image display unit 15 so as to display on the image display unit 15 the composite image IM3 generated in step S115. Once processing of step S117 is completed, processing progresses to step S119.

In step S119, the control unit 17 determines whether or not a stopping switch (switch for stopping execution of the imaging program) of the operation unit 10 has been turned on. When the stopping switch has been turned on, the control unit 17 stops execution of the imaging program, and when the stopping switch is off, processing returns to step S103.

In this manner, if the composite image IM3 is obtained (that is, if the imaging program is executed) by the imaging unit 1 according to the embodiment, a doctor is capable of accurately acquiring treatment position and therapeutic results of the subject ST (that is, progression of treatment) through observation of the composite image IM3. Furthermore, according to the embodiment, since the excitation light is made to pulse-light only for a duration corresponding to the imaging time (e.g., 16 msec) of one frame of the excitation light sensor 29 so as to acquire the fluorescent image IM2 in steps S109 to S113, irradiation with the excitation light is prevented from occurring over a long period of time, and treatment does not progress unintentionally. Supposing that total irradiation time of the excitation light to be emitted through a single photoimmunotherapy is 333 seconds, the excitation light to be emitted in steps S109 to S113 of the embodiment has 1/20,000 of the total irradiation energy, and it is thus considered that treatment does not progress; however, from the viewpoint of not letting treatment progress, pulse-lighting of the second light source 242 is preferably one time. However, irradiation may be carried out multiple times (that is, the fluorescent image IM2 is acquired multiple times) within a range that does not let treatment progress.

While the embodiment of the present invention has been described above, the present invention is not limited to the structure of the embodiment, and various modifications are possible within the range of the technical scope of the present invention.

For example, the embodiment is structured so as to make the excitation light pulse-light only for a duration corresponding to the imaging time (e.g., 16 msec) of one frame of the excitation light sensor 29 so as to acquire the fluorescent image IM2 in steps S109 to S113; however, the present invention is not limited to such a structure.

### <Modified Example 1>

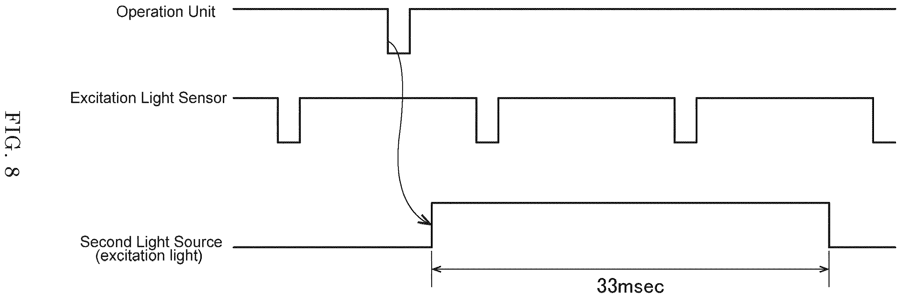

FIG. 8 is a timing chart illustrating a relationship between the timing of pulse-lighting of the second light source 242 according to the embodiment, timing of the operation of the excitation light switch (operation unit 10), and the frame rate of the excitation light sensor 29, as Modified Example 1 of the embodiment. Note that in FIG. 8, as in FIG. 7, the timing of when the excitation light switch is turned on is indicated on the operation unit 10 with a negative logic pulse signal, end of each field of the excitation light sensor 29 is indicated with a negative logic pulse signal, and timing of turning on the second light source 242 is indicated with a positive logic pulse signal. As illustrated in FIG. 8, this modified example differs from the structure of the embodiment in that the control unit 17 turns on the second light source 242 to pulse-light for 33 msec (that is, duration of two fields (one frame)) without synchronizing (that is, unsynchronized) with the field of the excitation light sensor 29 when it is determined in step S107 that the excitation light switch has been turned on. In this manner, a doctor is capable of accurately acquiring treatment position and therapeutic results of the subject ST (that is, progression of treatment) through observation of the composite image IM3 without unintentional progression of the treatment, even if the second light source 242 pulse-lights for 33 msec (that is, a duration of two fields (one frame)).

Furthermore, according to the embodiment, the light source 24 is described as including the first light source 241 that is a white light source and a second light source 242 that is an excitation light source; however, it is not limited to such structure.

### <Modified Example 2>

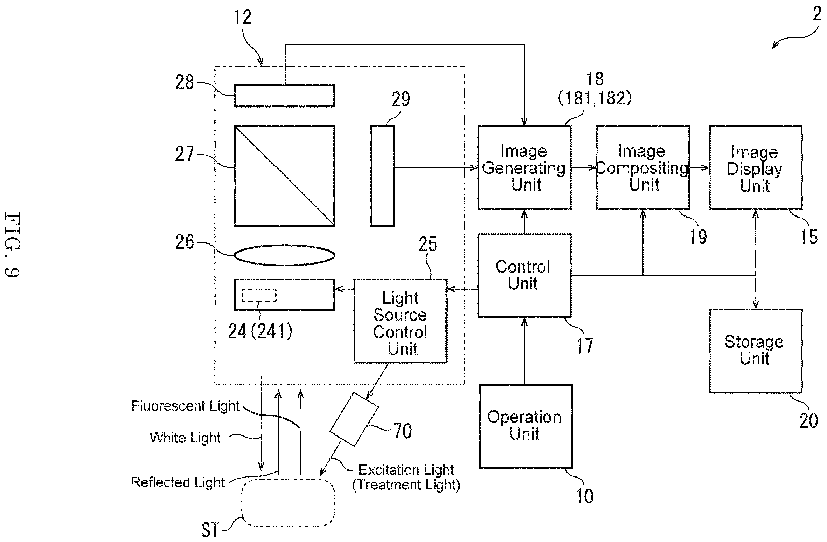

FIG. 9 is a block diagram illustrating a main control system of an imaging apparatus 2 according to Modified Example 2 of the embodiment. The imaging apparatus 2 of this modified example differs from the structure of the embodiment in that the light source 24 does not include the second light source 242, and that an excitation light source 70 in place of the second light source 242 is provided separately from the lighting and imaging unit 12. The excitation light source 70 of this modified example is also controlled by the light source control unit 25 in the same manner as is the second light source 242. In this manner, even when the excitation light source 70 is separate from the light source 24, a doctor is capable of accurately acquiring treatment position and therapeutic results of the subject ST (that is, progression of treatment) through observation of the composite image IM3. Note that aside from using the excitation light from the excitation light source 70 as a monitor light for acquiring treatment position and therapeutic results, the excitation light may also be used as a treatment light (that is, use the excitation light source 70 as a treatment light source). Further note that in this case, the imaging apparatus 2 may be used as a therapeutic apparatus.

The embodiment disclosed here is an example on all counts, and should be considered not limited hereto. The scope of the present invention is not limited to the description given above, and is intended to be provided within the scope of the patent claims, and include equivalent meaning to the scope of the patent claims and all modifications within the scope.

(Item 1) An imaging apparatus according to an aspect includes:
an excitation light source that irradiates a treating area of a subject, to which a treatment drug including the fluorescent pigment has been administered, with excitation light that excites a fluorescent pigment;
a first imaging unit that images the treating area and acquires a visible moving image;
a second imaging unit that acquires a fluorescent image by imaging fluorescent light generated from the fluorescent pigment of the treating area when irradiating with the excitation light;
a composite image generating unit that generates a composite image by superimposing the fluorescent image on the visible moving image; and
an image display unit that displays the composite image; wherein
the excitation light source irradiates with the excitation light having a predetermined pulse width.

According to the imaging apparatus of Item 1, a doctor is capable of accurately acquiring treatment position and therapeutic results of the subject (that is, progression of treatment) through observation of the composite image. Furthermore, since the excitation light is turned on to pulse-light, treatment does not progress unintentionally.

(Item 2) The imaging apparatus of Item 1, wherein
the predetermined pulse width is a duration of one field synchronized with the imaging timing of the second imaging unit.

According to the imaging apparatus of Item 2, a fluorescent image may be obtained using the minimum excitation light synchronized with the imaging timing of the second imaging unit.

(Item 3) The imaging apparatus of Item 2, wherein
the predetermined pulse width is approximately 16 ms.

According to the imaging apparatus of Item 3, a fluorescent image may be obtained within a duration of one NTSC field.

(Item 4) The imaging apparatus of Item 1, wherein
the predetermined pulse width is a duration of two fields unsynchronized with the imaging timing of the second imaging unit.

According to the imaging apparatus of Item 4, a fluorescent image may be obtained using the minimum excitation light unsynchronized with the imaging timing of the second imaging unit.

(Item 5) The imaging apparatus of Item 4, wherein
the predetermined pulse width is approximately 33 ms.

According to the imaging apparatus of Item 5, a fluorescent image may be obtained within a duration of two NTSC fields.

(Item 6) The imaging apparatus of any one of Item 1 to Item 5, wherein
the excitation light is a light of a wavelength 600 to 700 nm.

According to the imaging apparatus of Item 6, the fluorescent pigment can be reliably excited.

(Item 7) A therapeutic apparatus according to an aspect, includes:
the imaging apparatus of any one of Item 1 to Item 6; and
a treatment light source that irradiates the treating area with treatment light that excites the fluorescent pigment.

According to the therapeutic apparatus of Item 7, the subject may be treated using the imaging apparatus of any one of Item 1 to Item 5.

(Item 8) An imaging method according to an aspect, includes the steps of:
irradiating a treating area of a subject, to which a treatment drug including the fluorescent pigment has been administered, with an excitation light that excites a fluorescent pigment;
imaging the treating area and acquiring a visible moving image;
acquiring a fluorescent image by imaging fluorescent light generated from the fluorescent pigment of the treating area when irradiating with the excitation light;
generating a composite image by superimposing the fluorescent image on the visible moving image; and
displaying the composite image, wherein
the step of irradiating with an excitation light irradiates with the excitation light having a predetermined pulse width.

According to the imaging method of Item 8, a doctor is capable of accurately acquiring treatment position and therapeutic results of the subject (that is, progression of treatment) through observation of the composite image. Furthermore, since the excitation light is turned on to pulse-light, treatment does not progress unintentionally.

(Item 9) The imaging method of Item 8, wherein
the step of irradiating with the excitation light irradiates with the excitation light having the predetermined pulse width only one time, or irradiates in sync with a user's irradiation trigger.

According to the imaging method of Item 9, irradiation with only the minimum excitation light occurs, and treatment does not progress unintentionally.

(Item 10) The imaging method of either Item 8 or Item 9, wherein
the step of generating a composite image detects fluorescent light from the fluorescent image and superimposes the visible moving image on a resulting still image from the fluorescent light.

According to the imaging method of Item 10, a doctor is capable of accurately acquiring treatment position and therapeutic results of the subject (that is, progression of treatment) through observation of the composite image.

(Item 11) The imaging method of any one of Item 8 to Item 10, wherein the predetermined pulse width is a duration of one field synchronized with the imaging timing of the fluorescent image.

According to the imaging method of Item 11, a fluorescent image may be obtained using the minimum excitation light synchronized with the imaging timing of the fluorescent image.

(Item 12) The imaging method of Item 11, wherein
the predetermined pulse width is approximately 16 ms.

According to the imaging method of Item 12, a fluorescent image may be obtained within a duration of one NTSC field.

(Item 13) The imaging method of any one of Item 8 to Item 10, wherein
the predetermined pulse width is a duration of two fields unsynchronized with the imaging timing of the fluorescent image.

According to the imaging method of Item 13, a fluorescent image may be obtained using the minimum excitation light unsynchronized with the imaging timing of the fluorescent image.

(Item 14) The imaging method of Item 13, wherein
the predetermined pulse width is approximately 33 ms.

According to the imaging method of Item 14, a fluorescent image may be obtained within a duration of two NTSC fields.

(Item 15) The imaging method of any one of Item 8 to Item 14, wherein
the excitation light is a light of a wavelength 600 to 700 nm.

According to the imaging method of Item 15, the fluorescent pigment can be reliably excited.

### [Description of References]

1: Imaging apparatus
2: Imaging apparatus
10: Operation unit
11: Cart
12: Lighting and imaging unit
13: Wheel
14: Handle
15: Image display unit
16: Recess
17: Control unit
18: Image generating unit
19: Image compositing unit
20: Storage unit
24: Light source
25: Light source control unit
26: Zoom lens
27: Prism
28: White light sensor
29: Excitation light sensor
30: Arm mechanism
31: First arm member
32: Second arm member
33: Hinge
34: Hinge
35: Hinge
36: Column
37: Supporting part
41: Sub arm
42: Rotating shaft
43: Supporting part
70: Excitation light source
181: First imaging unit
182: Second imaging unit
241: First light source
242: Second light source
A: Reference character
B: Reference character
C: Reference character
IM1: Visible image
IM2: Fluorescent image
IM3: Composite image
ST: Subject

## Claims

1. An imaging apparatus, comprising:
an excitation light source that irradiates a treating area of a subject, to which a treatment drug including the fluorescent pigment has been administered, with excitation light that excites a fluorescent pigment;
a first imaging unit that images the treating area and acquires a visible moving image;
a second imaging unit that acquires a fluorescent image by imaging fluorescent light generated from the fluorescent pigment of the treating area when irradiating with the excitation light;
a composite image generating unit that generates a composite image by superimposing the fluorescent image on the visible moving image; and
an image display unit that displays the composite image; wherein
the excitation light source irradiates with the excitation light having a predetermined pulse width.

2. The imaging apparatus of claim 1, wherein the predetermined pulse width is a duration of one field synchronized with the imaging timing of the second imaging unit.

3. The imaging apparatus of claim 2, wherein the predetermined pulse width is approximately 16 ms.

4. The imaging apparatus of claim 1, wherein the predetermined pulse width is a duration of two fields unsynchronized with the imaging timing of the second imaging unit.

5. The imaging apparatus of claim 4, wherein the predetermined pulse width is approximately 33 ms.

6. The imaging apparatus of any one of claim 1 to claim 5, wherein the excitation light is a light of a wavelength 600 to 700 nm.

7. A therapeutic apparatus, comprising:
the imaging apparatus of any one of claim 1 to claim 6; and
a treatment light source that irradiates the treating area with treatment light that excites the fluorescent pigment.

8. An imaging method, comprising the steps of:
irradiating a treating area of a subject, to which a treatment drug including the fluorescent pigment has been administered, with an excitation light that excites a fluorescent pigment;
imaging the treating area and acquiring a visible moving image;
acquiring a fluorescent image by imaging fluorescent light generated from the fluorescent pigment of the treating area when irradiating with the excitation light;
generating a composite image by superimposing the fluorescent image on the visible moving image; and
displaying the composite image, wherein
the step of irradiating with an excitation light irradiates with the excitation light having a predetermined pulse width.

9. The imaging method of claim 8, wherein the step of irradiating with an excitation light irradiates with the excitation light having the predetermined pulse width only one time, or irradiates in sync with a user's irradiation trigger.

10. The imaging method of either claim 8 or claim 9, wherein the step of generating a composite image detects fluorescent light from the fluorescent image and superimposes the visible moving image on a resulting still image from the fluorescent light.

11. The imaging method of any one of claim 8 to claim 10, wherein the predetermined pulse width is a duration of one field synchronized with the imaging timing of the fluorescent image.

12. The imaging method of Claim 11, wherein the predetermined pulse width is approximately 16 ms.

13. The imaging method of any one of claim 8 to claim 10, wherein the predetermined pulse width is a duration of two fields unsynchronized with the imaging timing of the fluorescent image.

14. The imaging method of claim 13, wherein the predetermined pulse width is approximately 33 ms.

15. The imaging method of any one of claim 8 to claim 14, wherein the excitation light is a light of a wavelength 600 to 700 nm.
